# EUROPÄISCHE PATENTSCHRIFT

(11) **EP 0 737 747 B1**
(45) Veröffentlichungstag und Bekanntmachung des Hinweises auf die Patenterteilung: **11.06.2003**
(21) Anmeldenummer: 96103913.8
(22) Anmeldetag: 13.03.1996
(51) Int. Cl.: C12N 15/13, C12N 15/62, C12N 9/24

(54) **Cytoplasmatische Expression von Antikörpern, Antikörperfragmenten und Antikörperfragment-Fusionsmolekülen in E. coli**
Cytoplasmatic expression in E. coli of antibodies, antibody fragments and fusions thereof
Expression cytoplasmique dans E. coli des anticorps, fragments d'anticorps et fusions de ceux-ci

(30) Priorität: 11.04.1995 DE 19513676
(43) Veröffentlichungstag der Anmeldung: 16.10.1996
(73) Patentinhaber: HOECHST AKTIENGESELLSCHAFT, 65926 Frankfurt am Main (DE)
(72) Erfinder: Opper, Martin, Dr., D-35041 Marburg (DE); Bosslet, Klaus, Dr., D-35037 Marburg (DE); Czech, Jörg, Dr., D-35041 Marburg (DE)
(74) Vertreter: Fischer, Hans-Jürgen, Dr.

(56) Entgegenhaltungen:
- EP-A- 0 501 215
- EP-A- 0 590 530
- K. BOSSLET ET AL.: "Molecular and functional characterisation of a fusion protein suited for tumour specific prodrug activation." BRITISH JOURNAL OF CANCER, Bd. 65, Nr. 2, Februar 1992 (1992-02), Seiten 234-238, XP002092531 Basingstoke, Grossbritannien
- A. PLÜCKTHUN ET AL.: "Expression of functional antibody Fv and Fab fragments in Escherichia coli." METHODS IN ENZYMOLOGY, Bd. 178, 1989, Seiten 497-515, XP002024991 New York, NY, VSA
- M. RODRIGUES ET AL.: "Development of a humanized disulfide-stabilized anti-p185HER2 Fv-beta-lactamase fusion protein for activation of a cephalosporin doxorubici prodrug." CANCER RESEARCH, Bd. 55, Nr. 1, 1. Januar 1995 (1995-01-01), Seiten 63-70, XP002159150 Baltimore, MD, VSA
- H. HAISMA ET AL.: "A monoclonal antibody-beta-glucuronidase conjugate as activator of the prodrug epirubicin-glucuronide for specific treatment of cancer." BRITISH JOURNAL OF CANCER, Bd. 66, Nr. 3, September 1992 (1992-09), Seiten 474-478, XP000882039 Basingstoke, Grossbritannien
- A. DERMAN ET AL.: "Mutations that allow disulfide bond formation in the cytoplasm of Escherichia coli." SCIENCE, Bd. 262, Nr. 5140, 10. Dezember 1993 (1993-12-10), Seiten 1744-1747, XP002159151 Washington, DC, VSA
- M. HE ET AL.: "Functional expression of a dingle-chain anti-progesterone antibody fragment in the cytoplasm of a mutant Escherichia coli." NUCLEIC ACIDS RESEARCH, Bd. 23, Nr. 19, 11. Oktober 1995 (1995-10-11), Seiten 4009-4010, XP002159152 Oxford, Grossbritannien
- K. PROBA ET AL.: "Functional antibody single-chain fragments from the cytoplasm of Escherichia coli: influence of thioredoxin reductase (TrxB)." GENE, Bd. 159, Nr. 2, 4. Juli 1995 (1995-07-04), Seiten 203-207, XP004042207 Amsterdam, die Niederlande

### SEQUENCE LISTING

(1) GENERAL INFORMATION:
   (i) APPLICANT:
      (A) NAME: Behringwerke Aktiengesellschaft
      (B) STREET: Emil-von-Behring-Str. 76
      (C) CITY: Marburg
      (E) COUNTRY: Germany
      (F) POSTAL CODE (ZIP): 35041
      (G) TELEPHONE: 06421-39-2069
      (H) TELEFAX: 06421-39-4558
   (ii) TITLE OF INVENTION: Cytoplasmatische Expression von Antikoerpern,
      Antikoerperfragmenten und
      Antikoerperfragmentfusionsmolekuelen in E. coli
   (iii) NUMBER OF SEQUENCES: 7
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 30 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Enterobacteriaceae: Escherichia coli
   (vii) IMMEDIATE SOURCE:
      (B) CLONE: KS + E.c.-Beta-Gluc
   (xi) SEQUENCE DESCRIPTION: SEQ ID NO: 1:
   AAGCTTTCAT TGTTTGCCTC CCTGCTGCGG 30
(2) INFORMATION FOR SEQ ID NO: 2:
   (i) SEQUENCE CHARACTERISTICS:
      (A) LENGTH: 32 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Enterobacteriaceae: Escherichia coli
   (vii) IMMEDIATE SOURCE:
      (B) CLONE: KS + E.c.-Beta-Gluc
   (xi) SEQUENCE DESCRIPTION: SEQ ID NO: 2:
   TCTAGACCAT GGTACGTCCT GTACAAACCC CA 32
(2) INFORMATION FOR SEQ ID NO: 3:
   (i) SEQUENCE CHARACTERISTICS:
      (A) LENGTH: 57 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE: pAB Stop c-DNA HC/hu-Beta-Gluc
   (xi) SEQUENCE DESCRIPTION: SEQ ID NO: 3:
   GGATCCCATG GAACCAGAAC CAGAACCGAG CTCAACTCTC TTGTCCACCT TGGTGTT 57
(2) INFORMATION FOR SEQ ID NO: 4:
   (i) SEQUENCE CHARACTERISTICS:
      (A) LENGTH: 45 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE: pAB Stop c-DNA HC/hu-Beta-Gluc
   (xi) SEQUENCE DESCRIPTION: SEQ ID NO: 4:
   TCTAGATAAC GAGGGCAAAA AATGGAGGTC CAACTGCAGG AGAGC 45
(2) INFORMATION FOR SEQ ID NO: 5:
   (i) SEQUENCE CHARACTERISTICS:
      (A) LENGTH: 3169 base pairs
      (B) TYPE: nucleic acid
      (C) STRANDEDNESS: double
      (D) TOPOLOGY: circular
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Enterobacteriaceae: Escherichia coli
      (B) STRAIN: pRAJ210
   (vii) IMMEDIATE SOURCE:
      (B) CLONE: pTrc99 dicistr. Fab/E.c.-Beta-Gluc
   (ix) FEATURE:
      (A) NAME/KEY: CDS
      (B) LOCATION:3..641
   (ix) FEATURE:
      (A) NAME/KEY: CDS
      (B) LOCATION:666..3162
   (xi) SEQUENCE DESCRIPTION: SEQ ID NO: 5:
(2) INFORMATION FOR SEQ ID NO: 6:
   (i) SEQUENCE CHARACTERISTICS:
      (A) LENGTH: 213 amino acids
      (B) TYPE: amino acid
      (D) TOPOLOGY: linear
   (ii) MOLECULE TYPE: protein
   (xi) SEQUENCE DESCRIPTION: SEQ ID NO: 6:
   Met Asp Ile Gln Met Thr Gln Ser Pro Ser Ser Leu Ser Ala Ser Val
    1               5                  10                  15
   Gly Asp Arg Val Thr Ile Thr Cys Ser Thr Ser Ser Ser Val Ser Tyr
               20                  25                  30
   Met His Trp Tyr Gln Gln Lys Pro Gly Lys Ala Pro Lys Leu Leu Ile
           35                  40                  45
   Tyr Ser Thr Ser Asn Leu Ala Ser Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys His Gln Trp Ser Ser Tyr Pro Thr
                   85                  90                  95
   Phe Gly Gln Gly Thr Lys Val Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
(2) INFORMATION FOR SEQ ID NO: 7:
   (i) SEQUENCE CHARACTERISTICS:
      (A) LENGTH: 832 amino acids
      (B) TYPE: amino acid
      (D) TOPOLOGY: linear
   (ii) MOLECULE TYPE: protein
   (xi) SEQUENCE DESCRIPTION: SEQ ID NO: 7:

## Beschreibung

Die Erfindung betrifft die cytoplasmatische Expression von Antikörpern, Antikörperfragmenten und Antikörperfragmentfusionsmolekülen in E. coli. Insbesondere Antikörperfragmentfusionsmoleküle mit einem gegen Tumore gerichteten Antikörperteil und einem eine untoxische "Prodrug" in die toxische "Drug" spaltenden Enzymteil sind so unter Beibehaltung der jeweiligen funktionellen Eigenschaften günstig herstellbar.

Die Expression von Antikörpern oder Antikörperfragmenten unter Erhalt ihrer antigenbindenden Eigenschaft gelang bisher in E.coli nur unter Verwendung von Signalsequenzen, die den Transport ins Periplasma steuern. Die Expressionsausbeuten bewegen sich bei der periplasmatischen E.coli Expression im Bereich von wenigen µg/l Kulturmedium (Ayala et al., Bio Techniques 13, S. 790 - 799, 1992). Außerdem sind oft Rückfaltungsversuche notwendig, um funktionell aktive Antikörperfragmente (Fab) oder Antigenbinderegionen (sFv) zu gewinnen.

Es bestand daher weiterhin das Bedürfnis, bessere Methoden zur Expression von funktionell aktiven Antikörpern bzw. Antikörperfragmenten zu entwickeln. In Zusammenhang mit dem ADEPT Konzept (Bagshawe, Br.J. Cancer, Vol. 60, pp 275 - 281, 1989) sollten diese verbesserten Methoden auch auf Antikörperfragmentenzymfusionsmoleküle, speziell auf Fusionsproteine mit cytoplasmatischen Säuger- oder E.coli-Enzymen wie z. B. die E.coli β-Glucuronidase anwendbar sein.

Die β-Glucuronidase von Escherichia coli ist biochemisch und genetisch gut untersucht. Das Gen (uid A) wurde von Jefferson et al. (PNAS Vol. 83, pp 8447 - 8451, 1986) kloniert und als Reportergen für heterologe Kontrollregionen eingesetzt.

β-Glucuronidase (β-D-Glucuronisid Glucuronosohydrolase, E.C. 3.2.1.31) stellt eine saure Hydrolase dar, die die Spaltung von β-Glucuroniden katalysiert. Da die Säuger-Glucuronidasen intensiv untersucht wurden, sind vielerlei Substanzen für histologische, spektrophotometrische sowie fluorometrische Analysen verfügbar. Neue, zusätzliche Bedeutung hat dieses Enzym in der Verwendung für Fusionsproteine zur gezielten Tumortherapie erlangt. Dabei . wird die humane Glucuronidase in Form eines Fusionsproteins mit Antikörpern/Antikörperfragmenten bzw. Antigenbinderegionen verwendet (Bosslet et al., Br.J. Cancer, 65, 234 - 238, 1992). Alternativ zum humanen Enzym ist auch der Einsatz der homologen E.coli β-Glucuronidase möglich. Unter anderem hat die E.coll-β-Glucuronidase den Vorteil, daß ihre katalytische Aktivität bei physiologischem pH signifikant höher ist als die der humanen β-Glucuronidase.

Antikörperfragmentenzymfusionsmoleküle konnten bisher nur periplasmatisch in E.coli exprimiert werden. Der dabei verwendete Enzymanteil besteht daher stets aus periplasmatischen E.coli-Enzymen wie z. B. β-Lactamase (Goshorn et al., Canc.Res. 53, 2123 - 2117, 1993).

Die Expression eines Antikörperfragmentenzymfusionsmoleküls unter Verwendung eines cytoplasmatischen E.coli Enzyms wie der β-Glucuronidase war in funktionell aktivem Zustand, d. h. unter Beibehaltung von enzymatischer Aktivität - wie auch Antigenbindungsfähigkeit des Antikörperanteils - bisher nicht möglich. Die funktionell aktive Expression von den meisten Antikörpern bzw. Antikörperfragmentmolekülen erfordert in der Regel definierte Signalsequenzen zum Export der exprimierten Moleküle via endoplasmatischem Retikulum in das Kulturmedium (Animalzellen und Hefen) bzw. in das Periplasma (E.coli). Nur im endoplasmatischen Retikulum bzw. im Periplasma herrschen die notwendigen oxidativen Bedingungen zur Ausbildung der für die funktionelle Aktivität wichtigen Disulfidbrücken. Außerdem ist der sekretorische Syntheseweg oft für die korrekte dreidimensionale Faltung des exprimierten Proteins entscheidend.

Ein bezüglich Thioredoxinreduktase defizienter E.coli Stamm, z. B. der Stamm AD 494, kann Disulfidbrücken im Cytoplasma ausbilden und erlaubt so die intrazelluläre Expression von natürlicherweise sekretorischen Enzymen, wie z. B. alkalischer Phosphatase (Derman et al, Science, Vol. 262, 1744 -1747, 1993).

Es wurde nun gefunden, daß sich ein Fab-Molekül wie z. B. des MAK BW 431/26 funktionell aktiv, d. h. unter Beibehaltung der Antigenbindungseigenschaften in einem bezüglich Thioredoxinreduktase defizienten E.coli Stamm cytoplasmatisch exprimieren läßt, ebenso auch kompletter Antikörper.

Überraschenderweise konnte überdies ein Antikörperfragmentenzymfusionsmolekül bestehend aus beispielsweise dem cytoplasmatischen, nicht disulfidverbrückten E. coli-Enzym β-Glucuronidase und z.B. dem Fab BW 431/26, der seinerseits intramolekulare Cystinbrücken zur korrekten Faltung benötigt (Fab BW 431/26-E.coli-β-Glucuronidase) im Cytoplasma exprimiert und daraus in funktioneller Form isoiert werden. Dabei war das exprimierte Molekül löslich und keine Rückfaltungsversuche notwendig. Damit eröffnen sich neue Möglichkeiten zur wirtschaftlichen Produktion von Antikörpem, Antikörperfragmenten und Antikörperfragmentenzymfusionsmolekülen für die therapeutische und diagnostische Anwendung.

Die Erfindung betrifft daher Verfahren zur gentechnischen Expression von Antikörpern, Antikörperfragmenten oder Antikörperfragmentfusionsmolekülen mit cytoplasmatischen Säuger- oder E.coli-Enzymen als Fusionspartner mittels thioredoxinreduktase-defizienter E.coli Stämme und nachfolgender Isolierung der Expressionsprodukte aus dem Cytoplasma. Antikörperfragmente sind im Zusammenhang dieses Verfahrens bevorzugt Fab-Fragmente oder auch Antigenbinderegionen (sFv, Plückthun und Skerra, Meth. Enzymol. 178, S. 497-515, 1991) oder Antikörperfragmentenzymfusionsproteine mit cytoplasmatischen E.coli-Enzymen als Fusionspartner, ganz bevorzugt mit E.coli-β-Glucuronidase als Fusionspartner.

"Derman et al., Science 262, 1744-1745, 1993" offenbart die Verwendung verschiedener Thioredoxin-Reduktase defizienter E.coli Stämme zur Herstellung von Enzymen wie beispielsweise der alkalischen Phosphatase, β-Lactamase, β-Galaktosidase und Urokinase.

Die folgenden Beispiele erläutern die Erfindung.

### Beispiel 1

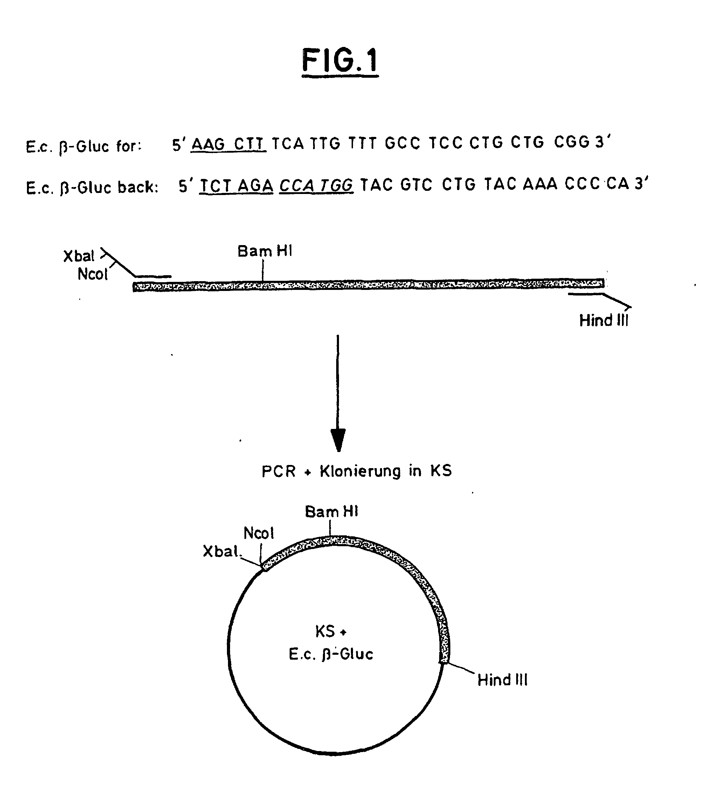

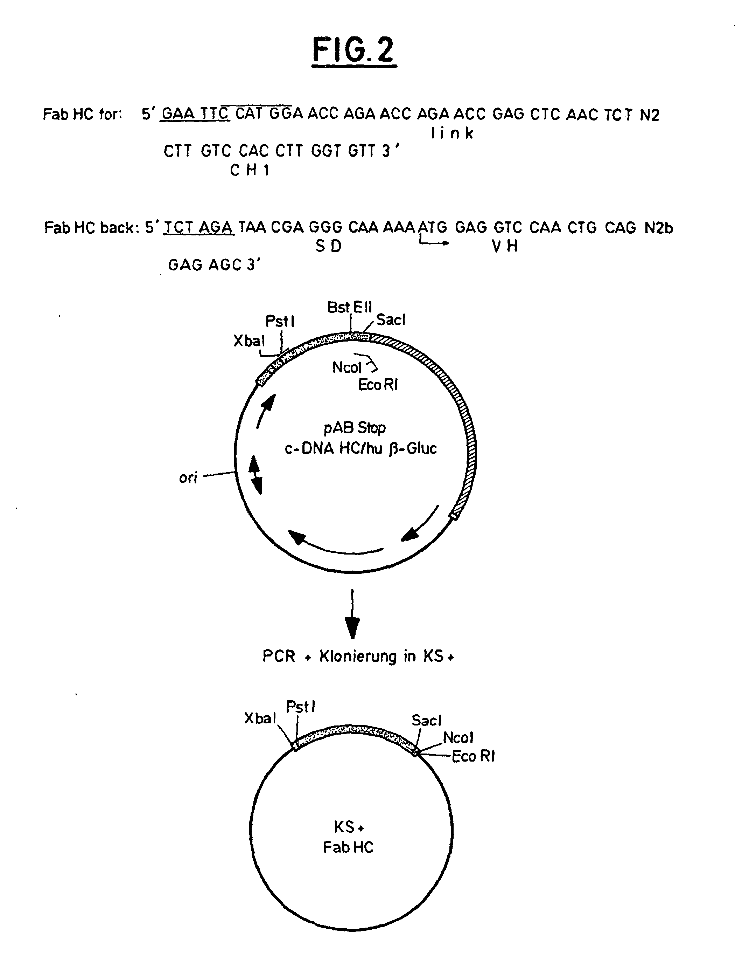

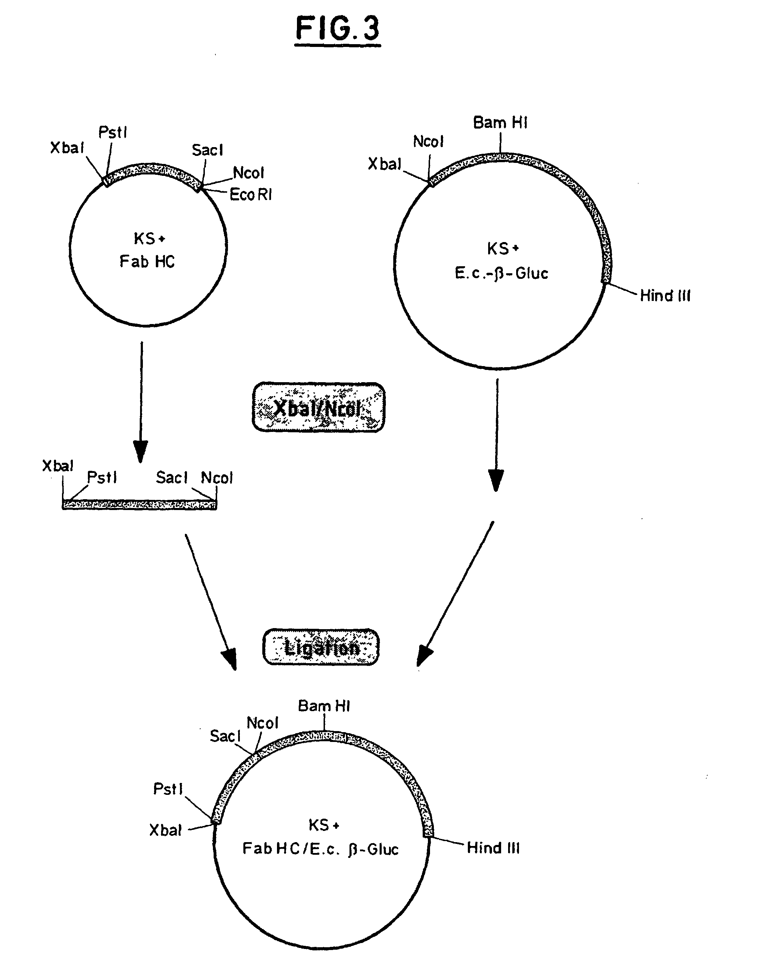

Konstruktion eines dicistronischen Expressionsvektors ohne Signalsequenzen
A) Klonierung der E.coli-β-Glucuronidase aus E.coli RR1:
   Mit den Primern E.c.β Gluc. for und E.c.β Gluc. back wurde die für E.coli β-. Glucuronidase kodierende DNA-Sequenz aus dem E.coli Stamm RR1 mittels PCR amplifiziert und über Xba I/Hind III in den Vektor KS kloniert (Fig. 1).
B) Klonierung von VH/CH1 und Linker(Verbindungsstück) MAK BW 431/26 vor E.coli-β-Glucuronidase:
   Mit den Primern Fab HC for und Fab HC back wurde aus einem vorhandenen cDNA-Konstrukt HC-hum-β-Glucuronidase mittels PCR die variable Domäne VH und die konstante Domäne CH1 amplifiziert und über Xba I/Eco RI in den Vektor KS kloniert (Fig. 2). Nach Verifizierung der DNA-Sequenz wurde ein über Xba I/Nco I Verdau gewonnenes DNA-Fragment in den mit XbaI/NcoI geschnittenen Vektor aus Klonierungsschritt A ligiert (Fig. 3).
C) Klonierung des Fab BW 431/26 E.coli-β-Glucuronidase in pTrc 99:

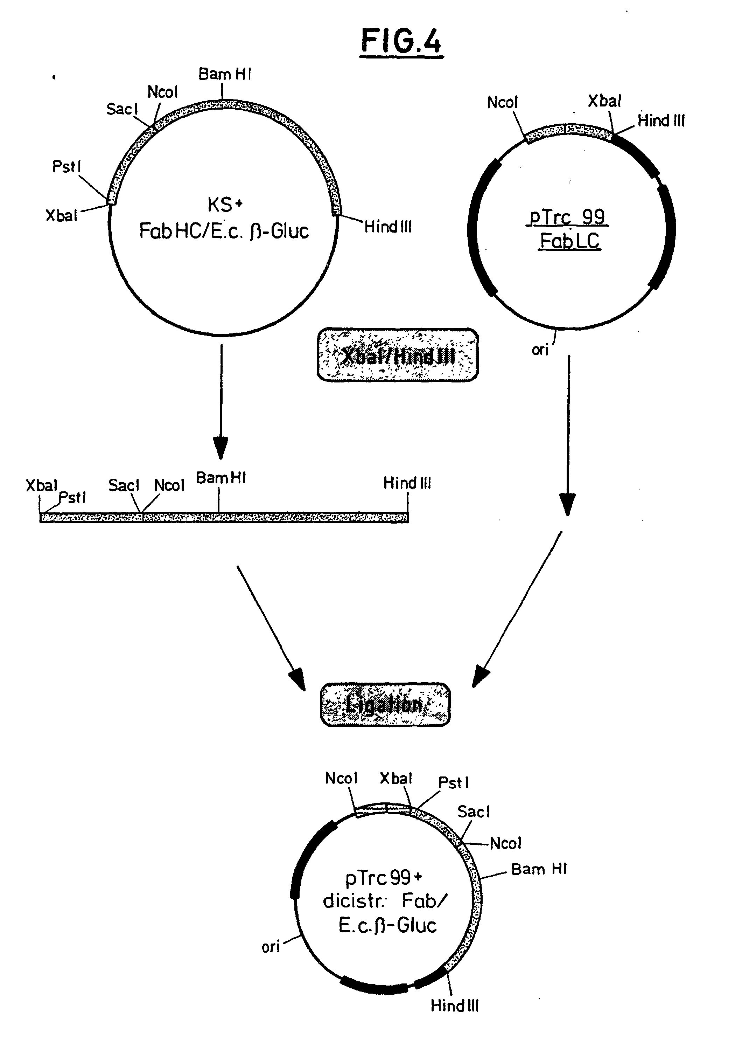

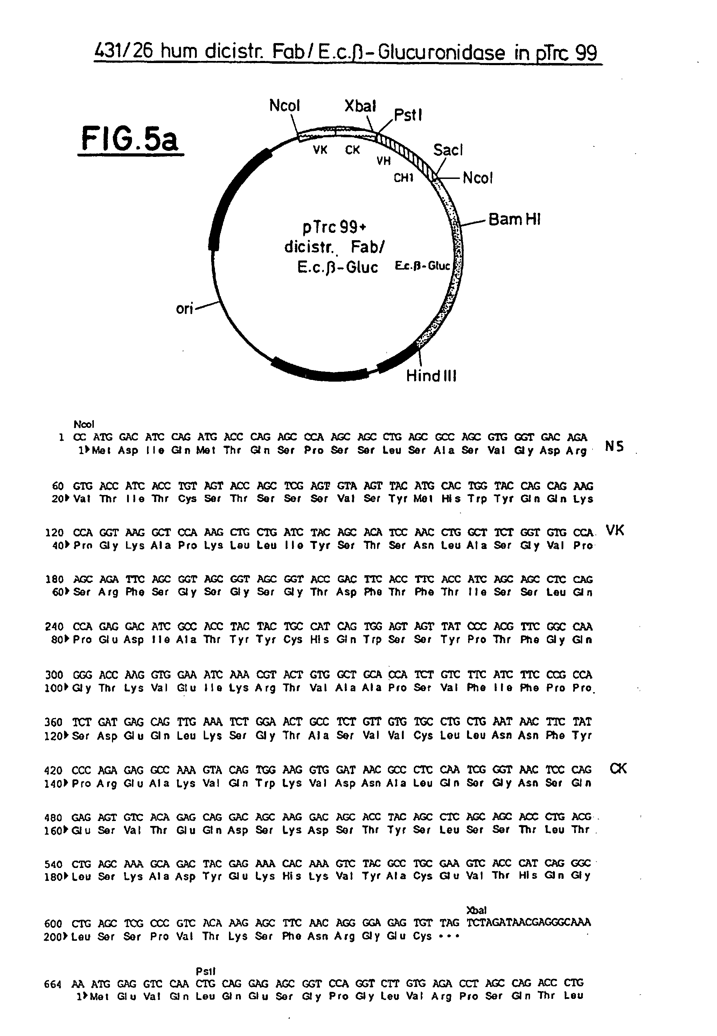

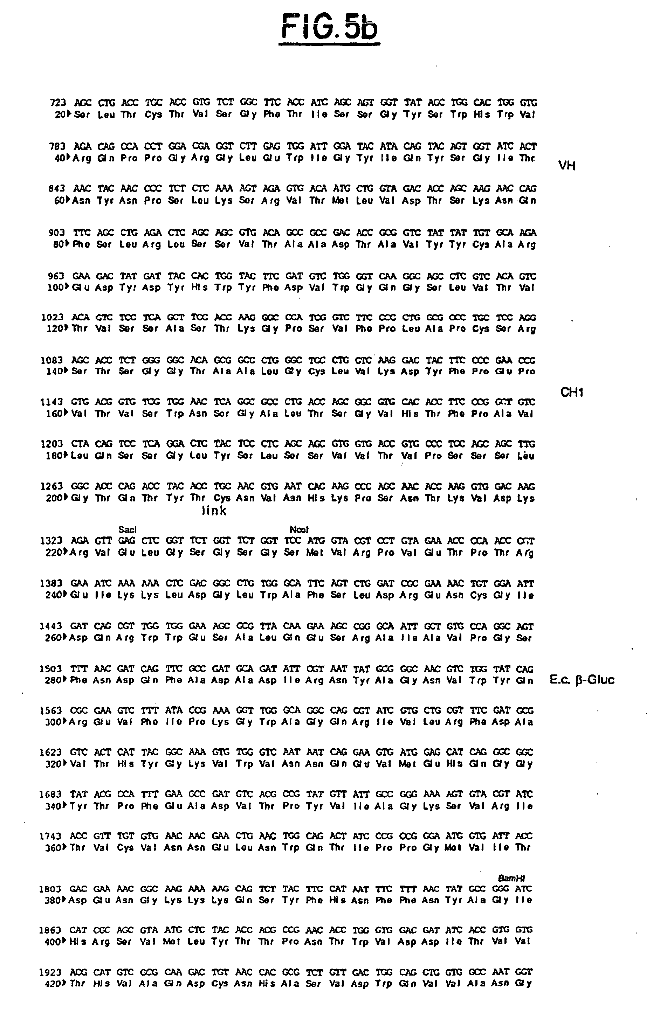

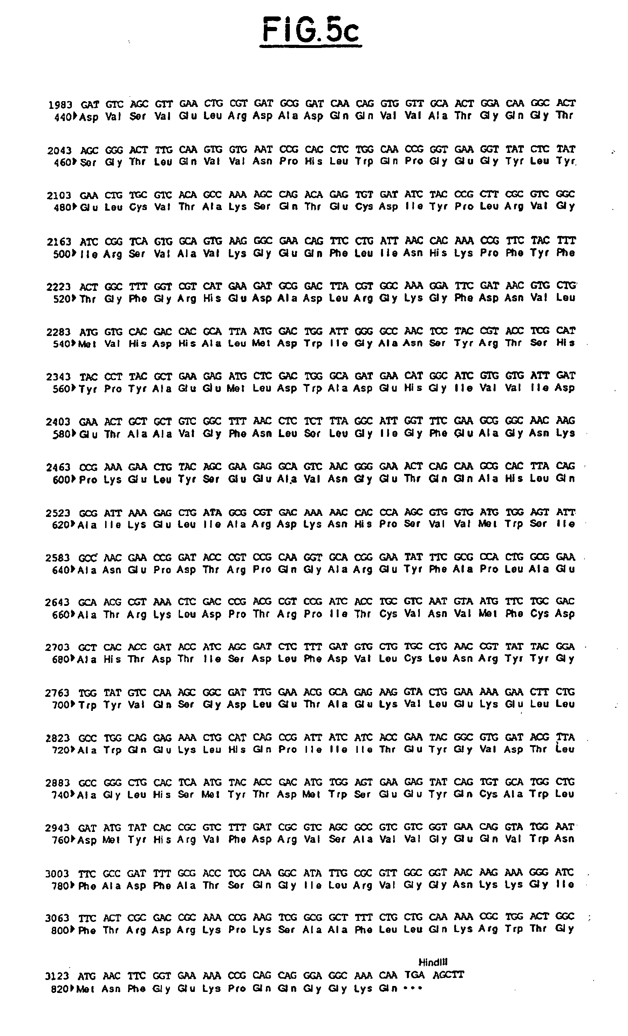

Zu der in dem Expressionsvektor pTrc99 (Amann et al, Gene 69, 301, 1988) vorhandenen leichten Kette des MAK BW 431/26 wurde ein Xba I/Hind III Fragment ligiert, das die korrespondierende schwere Kette fusioniert an die E.coli-β-Glucuronidase enthält (Fig. 4). Das entstandene Konstrukt hat die Struktur: Promotor (trc) - Shine Dalgarno Sequenz (SD) - VKICK MAK BW 431/26 - SD - VH/CH1 MAK BW 431/26 - E.coli-β-Gluc. - Transskriptions-Terminationssignal (Fig. 5).

### Beispiel 2

### Expression in AD 494

Übernacht-Kulturen des in Beispiel 1 beschriebenen pTrc dicistr. Fab-E.coli-β-Gluc. in AD 494 wurden 1:10 verdünnt und bei 25 °C bis zu einer OD₆₀₀ von 0,7 inkubiert. Nach Induktion mit 1mM IPTG für 19 - 22 Stunden wurden die Zellen 1-1,5 Stunden auf Eis inkubiert. Nach Pelletierung und Resuspendierung der Zellen in 10 ml PBS, pH 7,2 pro Liter Kulturvolumen in der French Press bei 1000 - 1500 Psi aufgebrochen. Der Zellaufbruch wurde bei 20.000 rpm im SS-34 Rotor geklärt und der Überstand für die weiteren Untersuchungen eingesetzt.

### Beispiel 3

### Affinitätschromatographische Reinigung des Fusionsmoleküls

Der über 2 µm Filter geklärte Zellaufbruchsüberstand wurde über die Bindung des Fusionsproteins an einen antiidiotypischen monoklonalen Antikörper (BW 2064(34) affinitätschromatographisch gereinigt (6 mg MAK/ml CnBr-aktivierter Sepharose 4B). Das an die Affinitätssäule gebundene Fusionsprotein wurde über pH-Wert Shift (pH 7,2 - pH 5,0) eluiert. Mittels Ultrafiltration erfolgte anschließend die Konzentrierung des Eluats (Filtron Macrosep. Omega NMWL:30 KD). Im SDS-PAGE wurden Zellaufbruchüberstand, Durchfluß, ankonzentriertes Eluat und Filtrat der Ultrafiltration analysiert. Die bei etwa 97 KD auftretende Bande entspricht molekulargewichtsmäßig dem erwarteten Fusionsprotein aus dem Schwerkettenanteil des Antikörpers und der E.coli Glucuronidase. Die bei etwa 70 KD auftretende Bande repräsentiert endogene β-Glucuronidase, die über die Bildung von Heterotetrameren (s. u.) zwischen exprimierter schwerer Kette/β- Glucuronidase und endogener β-Glucuronidase bei der Affinitäts-chromatographie mitgereinigt worden ist.

### Beispiel 4

### TSK-3000 Gelchromatographie

In der TSK 3000 Gelchromatographie wurde das native Molekül untersucht und das Molekulargewicht mit 450 KD bestimmt. Da die Glucuronidase im nativen. Zustand ein Tetramer bildet, entspricht das beobachtete dem theoretisch zu erwartenden Molekulargewicht. Im folgenden wird dieser Schritt im einzelnen beschrieben.

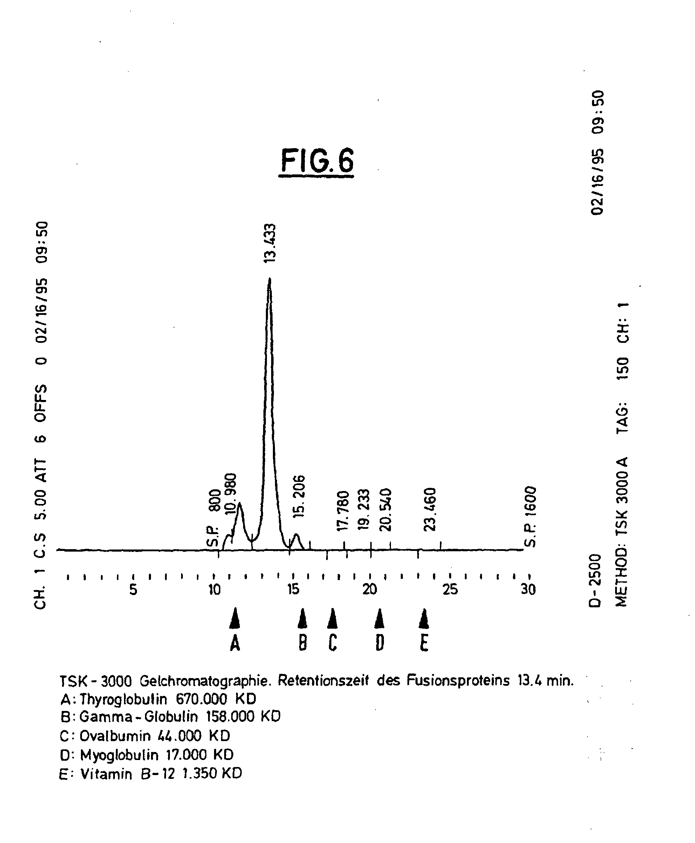

Von einem über Antiidiotyp affinitätsgereinigtem Fusionsprotein wurden 400 ng in 25 µl auf einer TSK Gel G 3000 SW XL Säule (TOSO HAAS, Best. Nr. 3.5WxN3211, 7,8 mm x 300 mm) in einem geeigneten Laufmittel (PBS, pH 7,2, 5 g/l Maltose, 4,2 g/l Arginin) mit einer Flußrate von 0,5 ml/min. chromatographiert. Die Merck Hitachi HPLC-Anlage (L-400 UV-Detektor, L-6210 Intelligent pump, D-2500 Chromato-Integrator) wurde mit etwa 20 bar betrieben, die optische Dichte des Eluats wurde bei 280 nm bestimmt und mittels eines LKB 2111 Multisac Fraktionssammlers 0,5 ml Fraktionen gesammelt, die anschließend im Spezifitätsenzymaktivitätstest (Beispiel 5) einer Analyse unterworfen wurden. Das Experiment ist in Fig. 6 dargestellt. Basierend auf den mittels Pfeilen markierten Molekulargewichtspositionen von Eichproteinen kann gefolgert werden, daß das funktionell aktive Fab-E.coli-β-Glucuronidase Fusionsprotein ein Molekulargewicht von etwa 450 KD besitzt.

### Beispiel 5

### Nachweis der Antigenbindungseigenschaften und der enzymatischen Aktivität

Die Fähigkeit des Fab-E.coli-β-Glucuronidase Fusionsproteins spezifisch an das durch den MAK 431/26 definierte Epitop auf CEA (Carcino Embryonales Antigen) zu binden und gleichzeitig die enzymatische Aktivität der β-Glucuronidase auszuüben, wurde in einem Spezifitäts-Enzymaktivitätstest gezeigt (EP-A-0 501 215 A2, Beispiel J). Der Test bestimmt die Freisetzung von 4-Methylumbelliferon aus 4-Methylumbelliferyl- β-Glucuronid durch den β-Glucuronidaseanteil des Fusionsproteins, nachdem das Fusionsprotein über den Fab-Anteil an das Antigen gebunden ist. Die ermittelten Fluoreszenzwerte werden als relative Fluoreszenzeinheiten (FE) angegeben (Tab. 1). Der Test zeigt eine signifikante Methylumbelliferon Freisetzung durch das Fusionsprotein bei den mit CEA beschichteten Platten.

Als Kontrolle dienten mit PEM (polymorphic epithelial mucin) beschichteten Platten. Das Fluoreszenzsignal war dort stets kleiner 30 FE.

**Tab. 1**

| | **1 : 2** | **1 : 4** | **1 : 8** | **1 : 16** | **1 : 32** | **1 : 64** | **1: 128** | **1: 256** |
|---|---|---|---|---|---|---|---|---|
| **FE Zellaufbruchsüberstand 1:3** | 8183 | 8149 | 7531 | 6631 | 4560 | 2509 | 1019 | 421,9 |
| **FE Durchfluß 1:1** | 6548 | 5231 | 3222 | 1477 | 525,2 | 214 | 86,19 | 46,29 |
| **FE Eluat pH 5,0 1:3** | 7782 | 7571 | 6360 | 4239 | 1983 | 815,7 | 302 | 113,9 |
| **FE Eluat pH 5,0 Ultrakonzent, 1:10** | 7904 | 8106 | 8036 | 7153 | 5802 | 3618 | 1651 | 665,7 |
| **FE Eluat pH 5,0 Ultrafiltrat 1:1** | 74,65 | 172,7 | 90,23 | 52,30 | 38,84 | 25,79 | 23,51 | 19,39 |

## Patentansprüche

1. Verfahren zur gentechnischen Expression von Antikörpern, Antikörperfragmenten oder Antikörperfragmentenzymfusionsmolekülen mit cytoplasmatischen Säuger- oder E. coil Enzymen als Fusionspartner, **dadurch gekennzeichnet, daß** Thioredoxinreduktase defiziente E. coli Stämme eingesetzt werden und die Expressionsprodukte aus dem Cytoplasma isoliert werden.

2. Verfahren nach Anspruch 1, **dadurch gekennzeichnet, daß** Fab-Fragmente oder Antigenbinderegionen (sFv) exprimiert werden.

3. Verfahren nach Anspruch 1, **dadurch gekennzeichnet, daß** das Antikörperfragmentenzymfusionsmolekül im Antikörper gegen Tumorzellen gerichtet ist und der Enzymteil in der Lage ist, untoxische "Prodrugs" in toxische "Drugs" zu spalten.

4. Verfahren nach Anspruch 3, **dadurch gekennzeichnet, daß** der Antikörperteil humanisiert wurde und der Enzymteil ein humanes cytoplasmatisches Enzym ist.

## Claims

1. A process for the recombinant expression of antibodies, antibody fragments or antibody fragment-enzyme fusion molecules containing cytoplasmic mammalian or E. coli enzymes as fusion partners, which comprises employing thioredoxin reductase-deficient E. coli strains and isolating the expression products from the cytoplasm.

2. The process as claimed in claim 1, wherein Fab fragments or antigen-binding regions (sFv) are expressed.

3. The process as claimed in claim 1, wherein the antibody fragment-enzyme fusion molecule in the antibody is directed against tumor cells and the enzyme moiety is able to cleave nontoxic prodrugs to give toxic drugs.

4. The process as claimed in claim 3, wherein the antibody moiety is humanized and the enzyme moiety is a human cytoplasmic enzyme.

## Revendications

1. Procédé pour l'expression par génie génétique d'anticorps, de fragments d'anticorps ou de molécules de fusion de fragments d'anticorps et d'enzymes avec des enzymes cytoplasmiques de mammifères ou de E.coli comme partenaires de fusion, **caractérisé en ce que** des souches de E. coli déficientes en thiorédoxine réductase sont utilisées et les produits d'expression sont isolés à partir du cytoplasme.

2. Procédé selon la revendication 1 **caractérisé en ce que** des fragments Fab ou des régions de liaison d'antigène (sFv) sont exprimés.

3. Procédé selon la revendication 1 **caractérisé en ce que** la molécule de fusion de fragment d'anticorps et d'enzyme est dirigée dans l'anticorps contre des cellules tumorales et la partie enzyme est en mesure de cliver des "prodrugs" non toxiques en "drugs" toxiques.

4. Procédé selon la revendication 3 **caractérisé en ce que** la partie anticorps a été humanisée et la partie enzyme est une enzyme cytoplasmique humaine.
